# EUROPEAN PATENT APPLICATION

(11) **EP 3 659 611 A2**
(43) Date of publication of application: **03.06.2020**
(21) Application number: 18838031.5
(22) Date of filing: 23.07.2018
(51) Int. Cl.: A61K 35/28, A61K 9/00

(54) **COMPOSITION FOR PREVENTING OR TREATING OSTEOPOROSIS CONTAINING EXOSOMES EXTRACTED FROM STEM CELLS AS ACTIVE INGREDIENT**

(30) Priority: 24.07.2017 KR 20170093503; 20.07.2018 KR 20180084760
(71) Applicant: Exostemtech Co., Ltd., Sangrok-gu Ansan-si, Gyeonggi-do 15588 (KR)
(72) Inventor: CHO, Yong Woo, Seongnam-si Gyeonggi-do 13530 (KR); LEE, Kyoung Soo, Suwon-si Gyeonggi-do 16534 (KR); CHOI, Ji Suk, Gunpo-si Gyeonggi-do 15804 (KR)
(74) Representative: Diehl & Partner GbR
(86) International application number: PCT/KR2018/008267
(87) International publication number: WO 2019/022451

(57) **Abstract**

The present invention relates to a pharmaceutical composition for preventing or treating osteoporosis comprising exosomes isolated from adipose tissue-derived stem cells as an active ingredient. The exosomes isolated from the adipose tissue-derived stem cells according to the present invention can facilitate osteogenesis and enhance bone density, and therefore it can be useful for prevention or treatment of osteoporosis.

## Description

### [TECHNICAL FIELD]

The present invention relates to a composition for preventing or treating osteoporosis comprising exosomes isolated from stem cells as an active ingredient.

### [BACKGROUND ART]

Various cells present in multicellular organisms including humans are known to secrete nanosized vesicles called 'exosomes'. Exosomes are vesicles of the same membrane structure as a cell membrane, and are known to play a role in transferring membrane components and protein RNA by defects in other cells and tissues. In particular, exosomes secreted from stem cells are known to help tissue regeneration, as they contain various growth factors and cytokines secreted by stem cells. In addition, exosomes can be safely used with equivalent effects of cell culture, since impurities such as cell waste, antibiotics, serum and the like in the cell culture are removed during the isolating process.

Bone tissue is a tissue which maintains homeostasis by sequentially causing osteolysis of osteoclasts and osteogenesis of osteoblasts. Osteoporosis is a disease in which the balance of the bone remodeling process is destroyed by various causes such as menopause, aging and the like, and the bone strength is weakened due to changes in bone microstructure. Osteoporosis is a disease of which prevention is very important, since it increases the risk of fractures such as spine, thighs and radius due to a decrease in bone density and it progresses slowly without any obvious symptoms.

The causes of osteoporosis are largely divided into postmenopausal osteoporosis (Type 1 osteoporosis) and senile osteoporosis (Type 2 osteoporosis), and the postmenopausal osteoporosis appears to be caused by rapid osteolysis with decreased estrogen secretion after menopause in women, and the senile osteoporosis appears by a decrease in bone density as bone absorption is increased than its formation with age

According to the statistics (National Statistical Office) on senior citizens in 2016, the population aged 65 and over in 2015 was 6.57 million, accounting for 13.2 % of the total population. As the elderly population increases, the number of osteoporosis patients classified as senile diseases is increasing rapidly. One in five (22.4 %) of adults aged 50 and over is osteoporosis, and one in two (47.9 %) is osteopenia. The prevalence of osteoporosis in the 70s and over is 68.5 % for women and 18.0 % in men. The prevalence of osteoporosis is expected to increase further higher, as Korea is rapidly entering an aging society in reality.

As a therapeutic agent for osteoporosis currently used clinically, osteolysis inhibitors such as bisphosphonate, calcitonin and the like, and osteogenesis promoters such as parathyroid hormone have been used. Estrogen administration is known to be effective in postmenopausal women. However, it has been reported that long-term administration of bisphosphonate preparations may cause side effects due to gastrointestinal disorders and excessive inhibition of bone remodeling, and also, long-term administration of estrogen increases atherosclerotic diseases such as venous thrombosis, myocardial infarction and the like and increases the breast cancer incidence.

Although patent applications for a therapeutic agent for osteoporosis using stem cells have been filed [Korean Patent Publication No. 2007-0101756, Korean Patent No. 679642, Korean Patent Publication No. 2014-0006323], there are problems in that adult stem cells are less efficient because of poor viability and compatibility during transplantation, and there is a risk that undifferentiated stem cells may form tumors. In addition, the stem cell culture may have remaining possibility of impurities such as cell waste, antibiotics and fetal bovine serum (FBS) and the like.

Accordingly, it is urgent to develop an osteoporosis therapeutic agent which has less side effects and can be administered in a long period and has excellent efficacy.

### [DISCLOSURE]

### [TECHNICAL PROBLEM]

Accordingly, to solve problems of the prior art, the present inventors have isolated exosomes containing an osteoclastogenesis inhibiting factor such as OPG (osteoprotegerin) from stem cells, and have continued researches to be applied for development a composition for preventing and preventing osteoporosis using thereof, thereby completing the present invention.

### [TECHNICAL SOLUTION]

Therefore, a purpose of the present invention is to provide a pharmaceutical composition for preventing or treating osteoporosis, comprising exosomes isolated from adipose tissue-derived stem cells as an active ingredient.

In addition, another purpose of the present invention is to provide an injectable preparation for preventing or treating osteoporosis, comprising exosomes isolated from adipose tissue-derived stem cells as an active ingredient.

Moreover, other purpose of the present invention is to provide a health functional food for preventing or improving osteoporosis, comprising exosomes isolated from adipose tissue-derived stem cells as an active ingredient.

Furthermore, other purpose of the present invention is to provide a use of exosomes isolated from adipose-derived stem cells in preparation of medicine for preventing or treating osteoporosis.

Additionally, other purpose of the present invention is to provide a method for preventing or treating osteoporosis comprising administering a pharmaceutical composition comprising exosomes isolated from adipose-derived stem cells as an active ingredient into a subject.

### [ADVANTAGEOUS EFFECTS]

The stem cell-derived exosome according to the present invention has an effect on prevention and/or treatment osteoporosis due to its excellent expression rate of bioactive factors which activate osteoblasts and inhibit the activity of osteoclasts. In addition, it can minimize side effects on conventional cell therapeutic agents, and can deliver effective substances stably and rapidly into cells as a cell-derived delivery system. Accordingly, the present invention can be applied as a composition for preventing and treating osteoporosis.

### [BRIEF DESCRIPTION OF THE DRAWINGS]

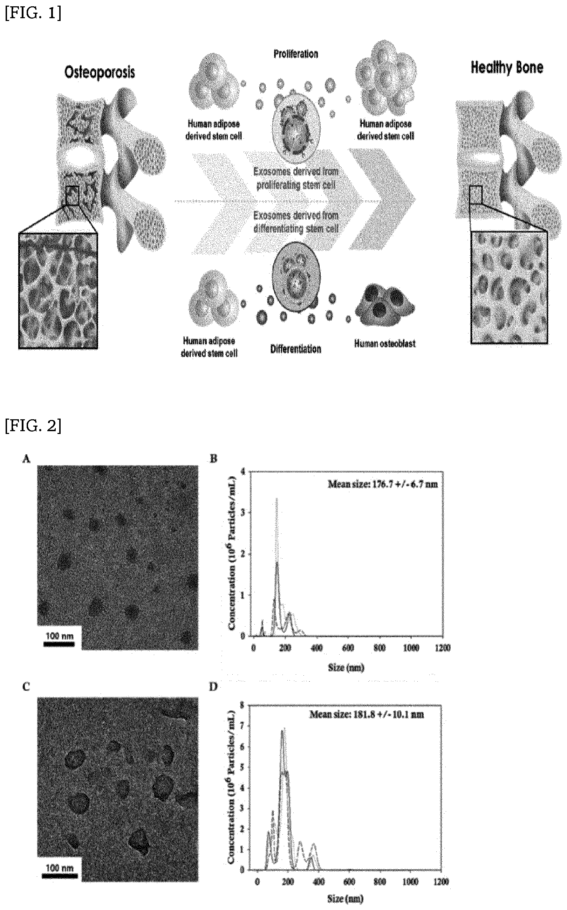

FIG. 1 is a mimetic diagram of the exosomes isolated from the proliferated human adipose-derived stem cells prepared in Example 1, exosomes isolated from the human adipose-derived stem cells that are differentiating into osteoblasts prepared in Example 2, and their applications.
FIG. 2 is a drawing which shows the result of analysis of properties of the exosomes isolated from the proliferated human adipose-derived stem cells prepared in Example 1, and the exosomes isolated from the human adipose-derived stem cells that are differentiating into osteoblasts prepared in Example 2, and shows the structure and shape of the exosomes confirmed using a transmission electron microscope and the size distribution of exosomes confirmed using a nanoparticle tracking analysis. (A) and (B) represent the structure, shape and size of exosomes isolated from proliferated human adipose-derived stem cell (ASC-EXO), and (C) and (D) represent them of exosomes isolated from differentiating human adipose-derived stem cells.

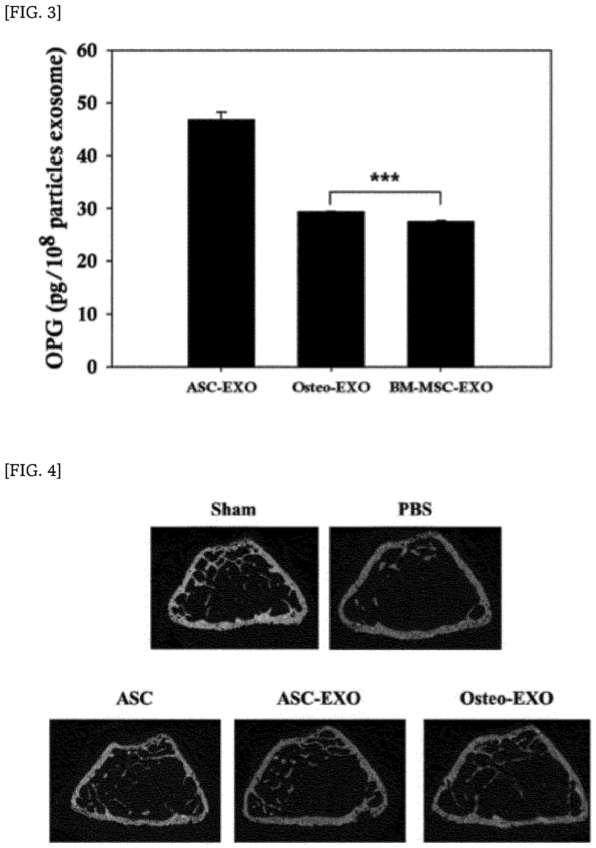

FIG. 3 is a drawing which shows the result of ELISA analysis on the content of OPG (osteoprotegerin) of exosomes isolated from proliferated human adipose-derived stem cells prepared in Example 1, and exosomes isolated from human adipose-derived stem cells which are differentiating into osteoblasts prepared in Example 2.
FIG. 4 is a picture photographing µCT by intravenously injecting exosomes isolated from proliferated human adipose-derived stem cells prepared in Example 1, and exosomes isolated from human adipose-derived stem cells which are differentiating into osteoblasts prepared in Example 2 (ASC-EXO, Osteo-EXO) into an osteoporosis-induced mouse model (ovariectomized mice model), and in 2 weeks after completing administration, removing a femur of the mouse.

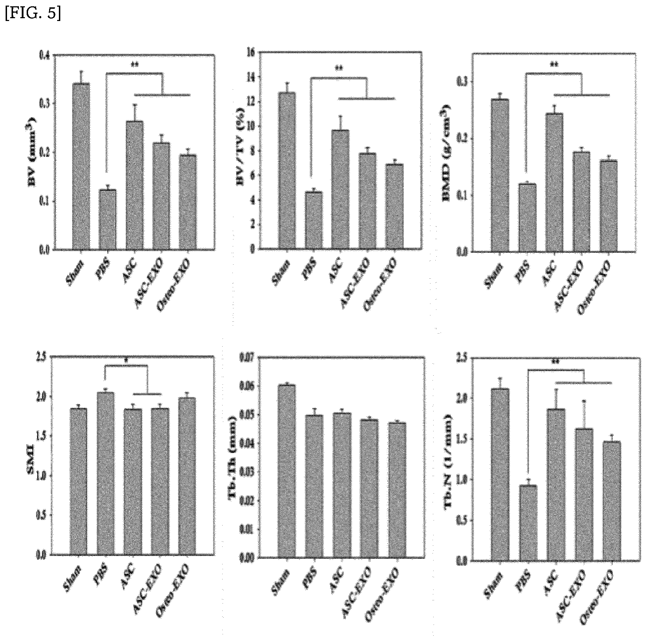

FIG. 5 shows the result of comparative analysis of parameters such as bone volume (BV), percent bone volume (BV/TV, tissue volume versus bone volume), bone mineral density (BMD), structure model index (SMI), tubercular thickness (Tb.Th), trabecular number (Tb.N) and the like, by photographing µCT by intravenously injecting exosomes isolated from proliferated human adipose-derived stem cells prepared in Example 1, and exosomes isolated from human adipose-derived stem cells which are differentiating into osteoblasts prepared in Example 2 (ASC-EXO, Osteo-EXO) into an osteoporosis-induced mouse model (ovariectomized mice model), and in 2 weeks after completing administration, removing a femur of the mouse.

### [BEST MODE]

To achieve the above purposes, the present invention provides a pharmaceutical composition for preventing or treating osteoporosis, comprising exosomes isolated from adipose tissue-derived stem cells as an active ingredient.

Herein, the term, "stem cell" has not only an autonomously replicating ability, but also a characteristic capable of differentiating into various cells by its multi-potency property, when an appropriate signal is provided if needed under the influence of the environment in which a cell is located, and is comprised in adipose, bone marrow, cord blood and placenta and the like. The stem cell of the present invention may be an autologous or allogenic derived stem cell, and may be derived from any type of animals including humans and non-human mammals.

Herein, the term, "adipose-derived stem cell" is a stem cell derived from adipose tissue, and the adipose tissue has a good condition for collecting stem cells, as it is easy to collect a large amount of tissues, and the adipose-derived stem cell may show stable growth and proliferation when culturing and differentiate into various cells when inducing differentiation.

The term used herein, "proliferated stem cell" may mean a stem cell that has been proliferated from a stem cell of passage 0 isolated from tissue to passage 7 using a general culture medium (Dulbecco Modified Eagle Medium, DMEM containing 10% fetal bovine serum, 1% penicillin/streptomycin). Therefrom, exosomes containing genetic information, proteins and growth factors of stem cells can be isolated.

The term used herein, "Osteogenic differentiating stem cells" means stem cells that are in the middle of differentiation from stem cells into osteoblasts, wherein the stem cells may be originated from bone cells. Therefrom, exosomes containing genetic information, proteins and growth factors related to differentiation into osteoblast can be isolated. Specifically, when the shape and properties of stem cells are changing during the stem cells are differentiating into osteoblasts, exosomes are isolated. Therefore, it is different from exosomes isolated from common stem cells.

The term used herein, "exosome" is a vesicle in a membrane structure which is secreted from various kinds of cells, and is known to play various roles such as delivering membrane components, proteins, and RNA by binding to other cells and tissue, and the like, and the average diameter of the exosome is approximately 30-200 nm.

The exosome isolated from the proliferated stem cell may have basic properties of stem cells, and may contain important growth factors, bioactive proteins and gene information, and the like, which are needed in tissue regeneration.

The exosomes isolated from osteogenic differentiating stem cells may have basic properties of stem cells, and may contain growth factors, various bioactive proteins and gene information and the like, which are important in bone differentiation. Specifically, it may comprise a factor related to prevention and treatment of osteoporosis, such as OPG (osteoprotegerin) which inhibits differentiation of pre-osteoclasts as a osteoclast activity inhibiting substance, BMPs (bone morphogenic proteins) which play a role of attracting surrounding mesenchymal stem cells to facilitate osteoblast differentiation as one of osteoblast activity promoting factors, and TGF-β (transforming growth factor-β) which facilitates migration of osteoprogenitor cells and differentiation into osteoblast as one of osteoblast activity promoting factors, and the like.

The exosomes may be prepared using exosome isolation methods known in the art, and for example,
it may use a method comprising; 1) proliferating a stem cell;
2) differentiating the proliferated stem cells into osteoblasts; and
3) isolating and purifying exosomes from the Osteogenic differentiating stem cells.

The "osteoblast differentiation induction" may mean inducing differentiation of a stem cell into an osteoblast.

The Osteogenic differentiating stem cell may be an adult-derived stem cell which can be differentiated into osteoblast. The adult-derived stem cell which can be differentiated into osteoblast may be a bone-marrow stem cell, a cord blood stem cell or an adipose-derived stem cell.

The adipose-derived stem cell may be a human or mammal-derived stem cell.

It has been confirmed that the exosomes isolated from an adipose-derived stem cell, and the exosomes isolated by the isolation method according to the present invention have an effect of preventing or treating osteoporosis.

Accordingly, the present invention includes a pharmaceutical composition for preventing or treating osteoporosis comprising an exosome isolated from an adipose-derived stem cell as an active ingredient.

The "composition for preventing or treating osteoporosis" according to the present invention is an effective substance for effectiveosteogenesis, and has distinction from conventional technologies in that it uses an exosome isolated from an osteogenic differentiating stem cell. Effectively sustainable regeneration of bone tissue is conducted by various growth factors related to proliferation and differentiation of cells supported in isolated and purified exosomes, and it may solve a problem of in vitro cell culture of conventional adult stem cells or a problem of calcification of tissue due to apoptosis, or the like.

The stem cell-derived exosome isolated during the period of differentiating into osteoblasts according to the present invention may deliver only active factors related to differentiation into osteoblasts as well as properties of stem cells, and therefore the same effect as the conventional treatment using stem cells while minimizing side effects.

The stem cell-derived exosome isolated during the period of differentiating into osteoblasts according to the present invention is nanosized vesicle secreted from cells. In addition, since it has a similar lipid structure to a cell membrane, effective regeneration induction of bone tissue is possible by excellent absorption rate to surrounding cells when injected in vivo and rapid delivery of effective substances.

The pharmaceutical composition for preventing or treating osteoporosis according to the present invention may comprise an exosome isolated from an adipose-derived stem cell in a pharmaceutically effective dose only, or may comprise one or more of pharmaceutically acceptable carriers, excipients, or diluents. The pharmaceutically effective dose means a sufficient amount for preventing, improving and treating symptoms of osteoporosis.

In addition, the "pharmaceutically acceptable" commonly means a composition which is physiologically accepted and does not cause an allergic reaction such as gastrointestinal disorders and dizziness, or a reaction similar thereto, when administered into humans.

The pharmaceutical composition may be various oral or parenteral formulations. In case of formulation, it is prepared using a commonly used diluent or excipient such as a filler, extender, binding agent, wetting agent, disintegrating agent, or surfactant.

Solid preparations for oral administration include tablets, pills, granules, capsules, and the like, and these solid preparations are prepared by mixing at least one or more of excipients, for example, starch, calcium carbonate, sucrose or lactose, gelatin or the like, to one or more of compounds. In addition, in addition to the simple excipient, lubricants such as magnesium stearate, talc and the like are used. Liquid preparations for oral administration include suspension, oral liquids, emulsion, syrup and the like, and it may comprise various excipients, for example, a wetting agent, sweetener, aroma, preservative or the like, in addition to a commonly used simple diluent, water, liquid paraffin. Preparations for parenteral administration include a sterile aqueous solution, non-aqueous liquid, suspension, emulsion, a lyophilized preparation and a suppository.

The pharmaceutical composition according to the specific example may use plant oil such as propylene glycol, polyethylene glycol, and olive oil, or injectable ester such as ethyl oleate, or the like, as the non-aqueous liquid and suspension. As the base of the suppository, witepsol, macrogol, tween 61, cacao butter, laurin butter, glycerogelatin, and the like may be used.

The pharmaceutical composition according to the specific example may be used as their pharmaceutically acceptable salts, and also, may be used alone or as an appropriate collection as well as binding to other pharmaceutically active compounds. The salt is not particularly limited unless it is pharmaceutically accepted, and for example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methane sulfonic acid, benzene sulfonic acid, toluene sulfonic acid, naphthalene sulfonic acid, or the like may be used.

The pharmaceutical composition according to the specific example may be parenterally administered or orally administered according to the purpose, and it may be administered once to several times so as to be administered in an amount of 0.1∼500 mg per body weight 1 kg daily. The dose for a specific patient may be changed according to the weight of the patient, age, gender, health condition, diet, administration time, administration method, excretion rate, severity of the disease, and the like.

The pharmaceutical composition according to the specific example may be used as formulated to oral formulations such as powder, granules, tablets, capsules, suspension, emulsion, syrup, aerosol and the like, external preparations such as ointment, cream and the like, and any types suitable for pharmaceutical preparations including a suppository and sterile injection solution, according to common methods, respectively.

The pharmaceutical composition according to the specific example may be administered by various routes such as parenteral or oral or the like into mammals such as rat, mouse, cattle, human and the like, and all the methods of administration may be expected, but preferably, it may be administered by oral, intrarectal, or intravenous, intramuscular, subcutaneous, intrauterine duramater or intracerebroventricular injection.

The pharmaceutical composition according to the specific example may further comprise a differentiation inducing substance such as dexamethasone, ascorbic acid and β-glycerophosphate and the like to differentiate a stem cell into osteoblasts, but not limited thereto.

In one example of the present invention, we determined the sizes of the exosomes isolated from adipose tissue-derived stem cells that have been proliferated (Adipose tissue-derived stem cell; ASC-EXO) and the exosomes isolated from adipose tissue-derived stem cells that are differentiating into osteoblasts (osteogenic differentiating stem cell-derived exosomes; Osteo-EXO), and found that each has average size of 50 to 200 nm.

In another example of the present invention, it has been confirmed that the effect of osteogenesis promotion and bone density enhancement is excellent when the composition comprising exosomes isolated from proliferated adipose tissue-derived stem cells (ASC-EXO) and exosomes isolated from adipose tissue-derived osteogenic differentiating stem cells (Osteo-EXO) is injected in vivo according to the present invention.

As other aspect, the present invention provides an injectable preparation for preventing or treating osteoporosis, comprising an exosome isolated from adipose tissue-derived stem cells as an active ingredient.

The injectable preparation may further comprise phosphate-buffered saline (PBS). In other words, the injectable preparation may be used by supporting exosomes isolated from adipose-derived stem cells in phosphate-buffered saline.

The injectable preparation may comprise hydrogel instead of phosphate-buffered saline.

The hydrogel may be any one selected from the group consisting of hyaluronic acid, gelatin, alginate, chitosan, fibrin, elastin, collagen and methyl cellulose, and specifically, it may be hyaluronic acid hydrogel, but not limited thereto.

The injectable preparation may comprise 1 x 10⁶ to 1 x 10¹¹ particles/mL or 1 x 10⁶ to 1 x 10¹¹ particles/day/body weight kg of exosomes, but not limited thereto.

The injectable preparation may be administered by injecting it into the damaged area such as bone or the like of mammals such as rat, mouse, cattle and human, and the like, and it may be intravenously administered.

The injectable preparation composition according to the present invention is easy to be injected in vivo, and therefore it is economical in the surgery time and cost aspects, and accordingly, it reduces pain of patients, aftereffects, and economic burden. In addition, since exosomes isolated when stem cells differentiate into osteoblasts comprise extracellular matrix derivatives and various growth factors related to proliferation and differentiation of cells, effective regeneration promotion and bond density enhancement of damaged bone tissue are possible. Therefore, a long-term effect may be expected by one-time procedure, and thus the conventional problem in that procedures should be conducted periodically in order to obtain the continuous effect.

As other aspect, the present invention provides a health functional food for preventing or improving osteoporosis, comprising an exosome isolated from adipose tissue-derived stem cells as an active ingredient. In other words, the health functional food according to the present invention may be used at the same time or respectively with medicine for treatment of osteoporosis, before or after the occurrence of osteoporosis, for preventing or improving osteoporosis.

The term used herein, "improvement" means all actions that at least reduce parameters associated with the condition being treated, for example, the degree of symptoms.

The health functional food composition according to the present invention may be added to a health supplement food such as food, beverages, and the like, on purpose of preventing or improving osteoporosis.

There is no particular limitation for kinds of the food. Examples of the food capable of adding the active ingredient include drinks, meat, sausage, bread, biscuit, rice cake, chocolate, candies, snacks, confectionery, pizza, ramen, other noodles, gum, dairy products including ice cream, various kinds of soup, beverages, alcoholic beverages and vitamin complexes, dairy products and processed dairy products, and the like, and they include all health functional foods in the common sense.

In the health functional food according to the present invention, the active ingredient may be added to the food as it is, or may be used with other food or food components, and may be appropriately used according to common methods. The mixing amount of the active ingredient may be determined appropriately according to its use purpose (for prevention or improvement). In general, in the preparation of food or beverages, the composition of the present invention is added in an amount of 15 % by weight or less, preferably, 10 % by weight or less, based on the raw material. However, in case of long period of intake for health and hygiene or for health control, the amount may be in the above range or less.

The composition for health beverages of the present invention has no particular limitation in other components, except for containing the active ingredient as a necessary component at the indicated ratio, and it may contain various flavoring agents or natural carbohydrates or the like as additional components as same as common beverages. Examples of the aforementioned natural carbohydrates include monosaccharides, for example, glucose, fructose, and the like; disaccharides, for example, maltose, sucrose, and the like; and polysaccharides, for example, common sugar such as dextrin, cyclodextrin, and sugar-alcohol such as xylitol, sorbitol, erythritol, and the like. As flavoring agents other than the above one, natural flavoring agents (thaumatin, stevia extracts (for example, rebaudioside A, glycyrrhizine, and the like) and synthetic flavoring agents (saccharine, aspartame, and the like) may be advantageously used. The ratio of the natural carbohydrates may be appropriately determined by selection of those skilled in the art.

The health functional food of the present invention other than the above one may contain various nutrients, vitamins, minerals (electrolyte), flavors such as synthetic flavor and natural flavor and the like, coloring agents and enhancers (cheese, chocolate, and the like), pectic acid and its salt, alginate and its salt, organic acid, protective colloid thickeners, pH adjusting agents, stabilizers, preservatives, glycerin, alcohol, carbonating agents used for carbonated beverages, and the like. These components may be used independently or in combination. The ratio of these additives may also be selected appropriately by those skilled in the art.

As other aspect, the present invention provides a use of an exosome isolated from an adipose-derived stem cell in preparation of medicine for preventing or treating osteoporosis.

The exosome isolated from the adipose-derived stem cell is as described above, and that it can be used as an active ingredient in a pharmaceutical composition for preventing or treating osteoporosis is as described above.

As other aspect, the present invention includes a method for preventing or treating osteoporosis comprising administering a pharmaceutical composition comprising an exosome isolated from an adipose-derived stem cell as an active ingredient to a subject in a therapeutically effective dose.

The subject means all animals including humans, who have or may have osteoporosis.

The term used herein, "therapeutically effective dose" means the amount of the active ingredient or pharmaceutical composition which induces a biological or medical reaction in tissue system, animals or humans considered by researchers, veterinarians, doctors, or other clinicians, and this includes the amount of inducing alleviation of symptoms of diseases or disorders to be treated. It is obvious that the therapeutically effective dose and the administration number of the active ingredient of the present invention will be changed according to the desired effect. Therefore, the optimal dose to be administered may be determined appropriately by those skilled in the art, and its range is various according to the kind of diseases, severity of diseases, active ingredient contained in the composition and content of other components, kind of formulations, patients' weight, age, gender, health condition, diet, administration time, administration method, excretion rate, and the like. The pharmaceutically effective dose of the exosome isolated from the adipose-derived stem cell according to the present invention may be 1 x 10⁶ to 1 x 10¹¹ particles/day/body weight kg. However, the pharmaceutically effective dose may be appropriately changed according to the degree of symptoms of osteoporosis, patients' age, body weight, health condition, gender, administration routes and treatment period and the like.

In the treatment method of the present invention, the composition comprising the exosome isolated from the adipose-derived stem cell of the present invention as an active ingredient may be orally administered or parenterally administered (for example, intravenously, subcutaneously, intraperitoneally or topically) according to the desired method.

Specifically, it may be administered to a subject by intravenous injection therapy, inhalation administration method, local administration method, or the like, and exosomes can be delivered to bone directly by inhalation and local administration methods, and also exosomes circulating along blood vessels during intravenous injection accumulate in bone naturally, and thus osteoporosis may be treated efficiently by the simple method as described above without complicated treatment processes.

Unless defined otherwise, all technical and scientific terms used herein have the same meaning as commonly understood by those skilled in the art to which this invention belongs. In general, the nomenclature used herein is well known and commonly used in the art.

### [MODE FOR INVENTION]

Hereinafter, preferable examples are provided to aid in understanding the present invention, but the following examples are intended to illustrate the present invention, and it is obvious that various changes and modifications are available within the scope and spirits of the present invention to those skilled in the art, and also, it is obvious that such changes and modifications fall within the appended claims.

### Example 1: Isolation of exosomes from proliferated human adipose-derived stem cells (ASC-EXO)

To isolate exosomes from proliferated stem cells, after culturing human adipose-derived stem cells (passage 3-7) in a general culture medium (Dulbecco Modified Eagle Medium, DMEM containing 10 % fetal bovine serum, 1 % penicillin/streptomycin), it was replaced with a serum-free and antibiotic-free DMEM medium without phenol red, to collect cell culture supernatant. From this, exosomes were isolated from proliferated human adipose-derived stem cells.

Specifically, the collected cell culture supernatant was centrifuged at 300 xg for 10 minutes to remove cells, and cell residues were removed using a filter with a pore size of 0.22 µm. Proteins in the solution collected after filtering were removed through a TFF (tangential flow filtration) process using a filter with 300 kD molecular weight cut off (MWCO). The TFF process was continuously repeated by adding saline buffer to the collected solution, and then exosomes were finally collected. The exosomes were kept frozen at -70 °C until use.

### Example 2: Isolation of exosomes from human adipose-derived stem cells which are differentiating into osteoblasts (Osteo-EXO)

In one example of the present invention, exosomes were isolated in the process of differentiating human adipose-derived stem cells into osteoblasts.

Specifically, human adipose-derived stem cells (passage 3-7) were cultured in osteoblasts differentiation medium (DMEM high-concentration glucose comprising 10% fetal bovine serum, 1% penicillin/streptomycin, 1 µM dexamethasone, 0.5 mM ascorbic acid, 0.01 M β-glycerophosphate (Dulbecco Modified Eagle Medium)), and once per 3 days, it was replaced with a serum-free and antibiotic-free DMEM medium without phenol red, and it was maintained for 24 hours. In 24 hours, the culture supernatant of differentiating stem cells was collected. After collecting the supernatant, a differentiation medium was added again to induce osteoblast differentiation, and this process was repeated for 3 weeks.

The collected cell culture supernatant was centrifuged at 300 xg for 5 minutes to remove cells. The collected cell culture supernatant was centrifuged at 300 xg for 10 minutes to remove cells, and cell residues were removed using a filter with a pore size of 0.22 µm. Proteins in the solution collected after filtering were removed through a TFF (tangential flow filtration) process using a filter with 300 kD molecular weight cut off (MWCO). The TFF process was continuously repeated by adding saline buffer to the collected solution, and then exosomes were finally collected. It was found that from after 1week of differentiation medium replacement, changes in cell shape occurred, and from after 2 weeks of differentiation medium replacement, calcium precipitates were formed, which are observed when differentiating into osteoblasts. Accordingly, exosomes were isolated from the supernatant collected during the period from 1 week to 4 weeks after inducing differentiation when the changes in cell shape were clearly shown.

### Example 3: Evaluation of properties of exosomes isolated from proliferated adipose-derived stem cells and exosomes isolated from adipose-derived stem cells differentiating into osteoblasts

The structure and shape of exosomes were determined using a transmission electron microscope and the size distribution of exosomes was determined using a nanoparticle tracking analysis for exosomes isolated from Example 1 and Example 2. The results were shown in FIG. 2.

(A) and (B) of FIG. 2 represent the structure, shape (TEM) and size (NTA) of exosomes isolated from proliferated human adipose-derived stem cells (ASC-EXO), and (C) and (D) represent them of exosomes isolated from human adipose-derived stem cells differentiating into osteoblasts (Osteo-EXO).

As the result of the transmission electron microscope ((A), (C) of FIG. 2), it was found that exosomes had a round nanoparticle shape, and the average sizes of these exosomes were ASC-EXO 176.7 nm and Osteo-EXO 181.8 nm ((B), (D) of FIG. 2).

### Example 4: Evaluation of OPG (Osteoprotegerin) expression

OPG expression in exosomes of 1 x 10⁸particles/mL of exosomes isolated in Example 1 and Example 2 of the present invention was quantitatively analyzed using a human osteoprotegerin ELISA Kit. Then, exosomes isolated from bone-marrow mesenchymal stem cells (BM-MSC-EXO) were used as a comparative control group.

As a result, exosomes isolated from proliferated stem cells (ASC-EXO) showed the significantly high OPG content compared to exosomes isolated from bone-marrow-derived stem cells (BM-MSC-EXO) (46.79 pg/10⁸ particle exosomes). It was found that the OPG content of exosomes isolated from human adipose-derived stem cells differentiating into osteoblasts (Osteo-EXO) was 29.38 pg/10⁸ particle exosomes, and there was a difference at 0.001 significant level compared to the OPG content of exosomes isolated from bone-marrow-derived stem cells (BM-MSC-EXO) (27.44 pg/10⁸ particle exosomes) (P < 0.001} [See FIG. 3]

### Example 5: Evaluation of osteogenesis facilitation and bone density enhancement

To confirm osteogenesis and bone density enhancement using exosomes, proliferated stem cell exosomes (ASC-EXO) and exosomes isolated from stem cells differentiating into osteoblasts (Osteo-EXO) were intravenously administered into an osteoporosis-induced mouse (ovariectomized mice model) 6 times for 2 weeks at a particle concentration of 1x10⁸ particles/mL. PBS was intravenously administered 6 times for 2 weeks as a negative control group. The adipose-derived stem cells (ASC) were intravenously administered twice, 5x10⁵ each as a positive control group, and a sham group who underwent open surgery was also analyzed.

In 2 weeks after completing administration, parameters such as bone volume (BV), percent bone volume (BV/TV, tissue volume versus bone volume), bone mineral density (BMD), structure model index (SMI), tubercular thickness (Tb.Th), trabecular number (Tb.N) and the like were compared and analyzed by removing thighs of the mouse and photographing µCT.

The result of photographing µCT was shown in FIG. 4. In addition, the result of comparative analysis of parameters such as bone volume (BV), percent bone volume (BV/TV, tissue volume versus bone volume), bone mineral density (BMD), structure model index (SMI), tubercular thickness (Tb.Th), trabecular number (Tb.N) and the like was as FIG. 5.

It was found that the bone volume (BV), percent bone volume (BV/TV), bone density (BMD) and trabecular number (Tb.N) increased in a 0.01 significant level compared to the negative control group (PBS), although the numerical value of the tubercular thickness (Tb.Th) did not exhibit a significant difference in ASC, ASC-EXO and Osteo-EXO administration groups compared to the negative control group (PBS). In addition, it was found that the structure model index (SMI) analyzing the cancellous structure was reduced in ASC and ASC-EXO administration groups compared to the negative control group (PBS) in a 0.05 significant level.

(SMI is a structure model index and shows a pattern of increasing the index in old age or when having a disease such as osteoporosis. It was found that exosomes alleviated osteoporosis through the result of significant reduction when administering exosomes, compared to the negative control group. The Tb.Th numerical value means the tubercular bone thickness, and shows a tendency of reduced thickness in an osteoporosis model. In the present technology, there was no difference in TbTh, but there were significant differences in other bone volume (BV), percent bone volume (BV/TV, tissue volume versus bone volume), bone mineral density (BMD), tubercular thickness (Tb.Th) and cancellous structure index (SMI) when treating ASC-EXO and Osteo-EXO, and therefore, it is considered that they are effective regarding osteoporosis.)

## Claims

1. A pharmaceutical composition for preventing or treating osteoporosis, comprising exosomes isolated from adipose tissue-derived stem cells as an active ingredient.

2. The pharmaceutical composition according to claim 1,
wherein the exosomes are isolated from proliferated adipose tissue-derived stem cells.

3. The pharmaceutical composition according to claim 1,
wherein the exosomes are isolated from adipose tissue-derived stem cells that are differentiating into osteoblasts.

4. The pharmaceutical composition according to claim 1,
wherein the adipose tissue-derived stem cells are human or mammal-derived stem cells.

5. An injectable preparation for preventing or treating osteoporosis, comprising exosomes isolated from adipose tissue-derived stem cells as an active ingredient.

6. The injectable preparation according to claim 5,
wherein the injectable preparation comprises the exosomes at a concentration of 1 x 10⁶ to 1 x 10¹¹ particles/mL.

7. A health functional food for preventing or improving osteoporosis, comprising exosomes isolated from adipose tissue-derived stem cells as an active ingredient.

8. The health functional food according to claim 7,
wherein the exosomes are isolated from proliferated adipose tissue-derived stem cells.

9. The health functional food according to claim 7,
wherein the exosomes are isolated from adipose tissue-derived stem cells that are differentiating into osteoblasts.

10. A use of exosomes isolated from adipose-derived stem cells in preparation of medicine for preventing or treating osteoporosis.

11. A method for preventing or treating osteoporosis comprising administering a pharmaceutical composition comprising exosomes isolated from adipose-derived stem cells as an active ingredient into a subject in a therapeutically effective amount.

12. The method according to claim 11,
wherein the exosomes are isolated from proliferated adipose tissue-derived stem cells.

13. The method according to claim 11,
wherein the exosomes are isolated from adipose tissue-derived stem cells that are differentiating into osteoblasts.

14. The method according to claim 11,
wherein the adipose tissue-derived stem cells are human or mammal-derived stem cells.
